# EUROPEAN PATENT APPLICATION

(11) **EP 2 711 437 A1**
(43) Date of publication of application: **26.03.2014**
(21) Application number: 12199196.2
(22) Date of filing: 21.12.2012
(51) Int. Cl.: C12R 1/645, A01N 63/04, C12N 1/14

(54) **Treatments of powdery mildews in plants**

(30) Priority: 19.09.2012 IT VI20120228
(71) Applicant: Fondazione Edmund Mach, 38010 San Michele all'Adige, TN (IT)
(72) Inventor: The designation of the inventor has not yet been filed
(74) Representative: Lahrtz, Fritz

(57) **Abstract**

The present invention, among others, relates to fungal strain *Ampelomyces quisqualis* Fem307 and variants thereof, and to preparations thereof, as well as to related uses and methods for treating powdery mildews in plants. It also relates to uses or methods for treating powdery mildews in plants that involve one or more activators.

## Description

### FIELD OF THE INVENTION

The present invention relates to the field of biofungicides, particularly to biocontrol of powdery mildew in plants. Certain aspects relate to a novel strain of *Ampelomyces quisqualis,* uses thereof and methods employing same.

### BACKGROUND OF THE INVENTION

Powdery mildew is a well-known fungal disease that affects a wide variety of plants. It is caused by many different species of fungi, mainly from the order *Erysiphales.*

The fungus *Ampelomyces quisqualis* is a hyperparasite of powdery mildews. More specifically, it is a specific mycoparasite of such fungi in the order *Erysiphales,* and is the most studied biocontrol agent of powdery mildews. Several *A. quisqualis* strains are available from culture collections and one strain isolated in Israel (CNCM I-807) has been formulated, registered and commercialized under the trade name of AQ10 (Kiss 1997; Sztejnberg 1993). Many molecular analyses based on the internal transcribed spacer (ITS) region of the nuclear ribosomal RNA gene (nrDNA) have revealed genetic diversity among *A. quisqualis* strains (Kiss et al. 2011; Liang et al. 2007; Park at al. 2010). Commercial strain AQ10 is widely exploited to control powdery mildew of various crops. Report data on the effectiveness in the powdery mildew control by AQ10 application are contradictory. In some experiments, a very good control of powdery mildews of various crops was achieved, but some other trials showed the biocontrol level was unsatisfactory, although parasitism of powdery mildew colonies on the treated crops did occur (cf. e.g. Gilardi et al. 2012). Some inconsistency on the level of efficacy of *A. quisqualis* under field conditions is also reported. However a number of examples of acceptable disease control have been reported for greenhouse and field-grown vegetable crops (Sztejnberg 1993). Repeated applications are generally preferred and high humidity and rainfall aid in spread to developing mycoparasite. However, there is still a considerable interest in finding more effective strains within *A. quisqualis* specie, which differ considerably with respect to their biocontrol efficacy. A group of strains isolated from different fungal host and plants were identified as highly aggressive in reducing the number of *P. xanthii* conidia.

Despite extensive research and progress made so far, powdery mildew infections remain among the most important plant pathologies in this field. Accordingly, there remains a need in the art for alternative or improved, respectively, treatments of powdery mildews in plants. This is particularly so in view of the various well-known advantages of biofungicides such as *A. quisqualis* when compared to standard fungicides. That is to say, *A. quisqualis* strains are generally considered non pathogenic and non infectious to humans and animals. As one example, even though strain *A. quisqualis* AQ10 has been used since 1995 in the United States, the present inventors are unaware of a report on undesirable effects thereof on agricultural personnel.

The present inventors have succeeded in finding a new effective strain of *A. quisqualis,* which can be used in the field to control powdery mildews. Moreover, with the present studies, the present inventors have surprisingly linked increased germination of *A. quisqualis* with enhanced biocontrol. Furthermore, the present inventors have surprisingly enhanced the efficiency of *A. quisqualis,* and particularly also of the above strain, in the biological control of powdery mildews by increasing the conidial germination rate of the fungus by using an activator.

### SHORT DESCRIPTION OF THE FIGURES

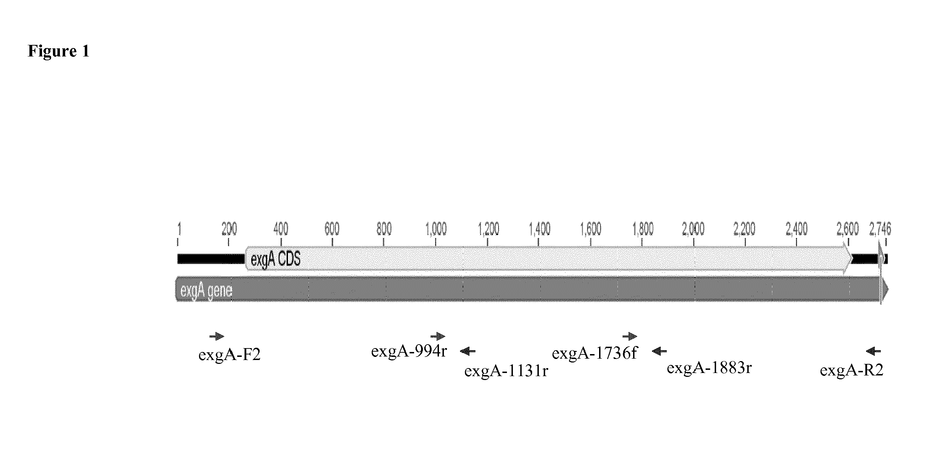

**Figure 1****:** This figure depicts the strategy used to sequence the exgA gene.

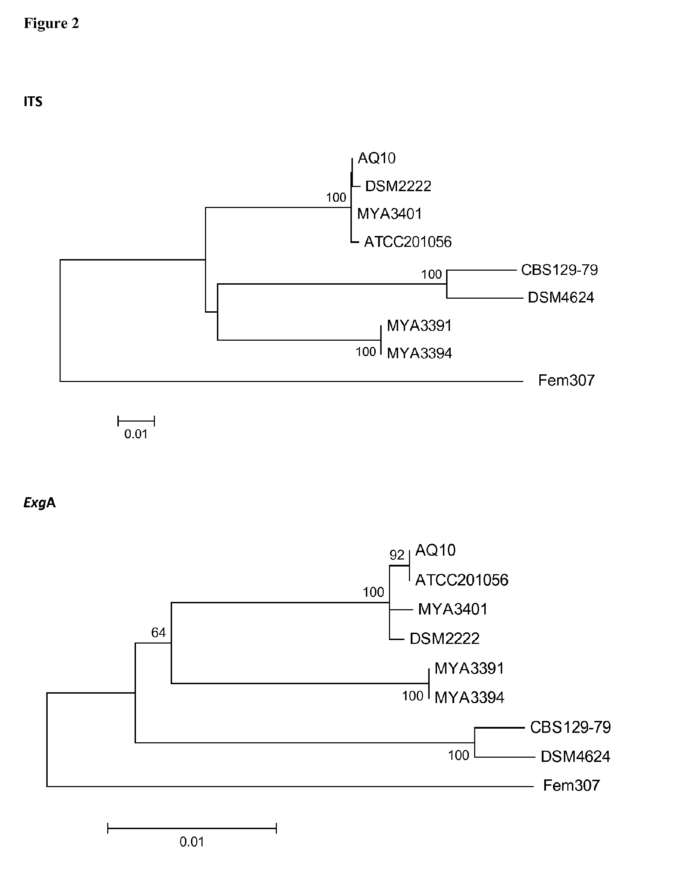

**Figure 2****:** This figure depicts Phylogenetic relationships of nine *A. quisqualis* strains based on ITS and *exg*A. The trees are midpoint rooted and inferred using maximum likelihood (ML). ML bootstrap support values inferred from 100 replicates are also presented.

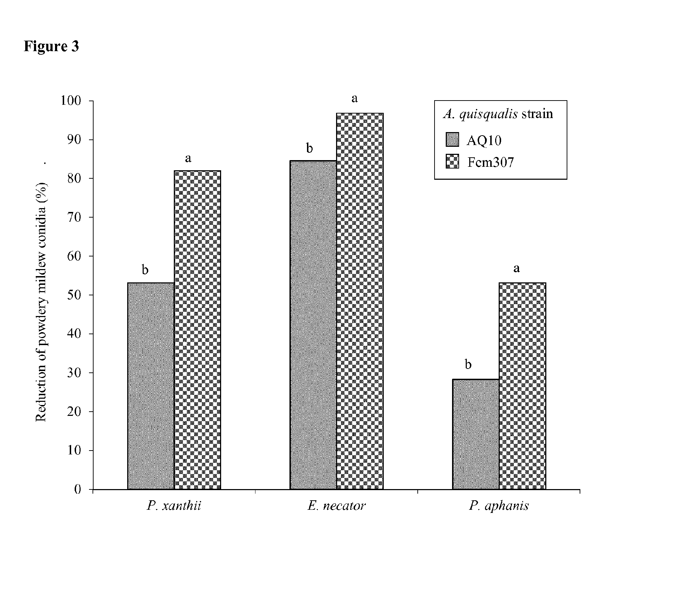

**Figure 3****:** This figure illustrates reduction in the number of powdery mildew conidia of P. *xanthii, E. necator* and *P. aphanis* in comparison with untreated control on leaves sprayed with Fem307 and AQ10. Columns with the same design represent the same *A. quisqualis* strain. Columns with the same letters (a-c) do not significantly differ (P ≤ 0.05, Tukey's test). Presented values are means of nine replicates derived from three independent experiments with three replicates per experiment

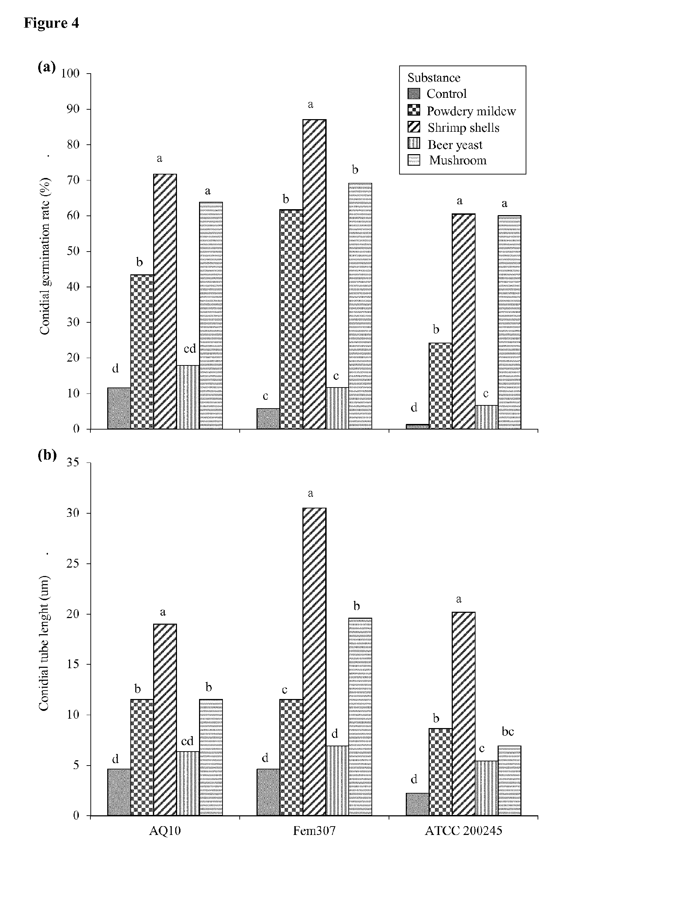

**Figure 4****:** This figure shows effects of different substances (Table 2) on the conidial germination (%) (a) and tube elongation (µm) (b) of AQ10, Fem307 and ATCC 200245. Measurements were taken in aqueous suspensions containing mixtures of *A. quisqualis* conidia and substances that had been incubated together for 24 h at 25°C. Untreated conidia were used as control. Columns with the same design represent the same *A. quisqualis* strain. Columns labeled with the same letter (a-c) are not significantly different (P ≤ 0.05) according to Tukey's test. Three replicates (50 conidia per replicate) of each strain were evaluated. Presented values are means of three independent experiments with three replicates per experiment.

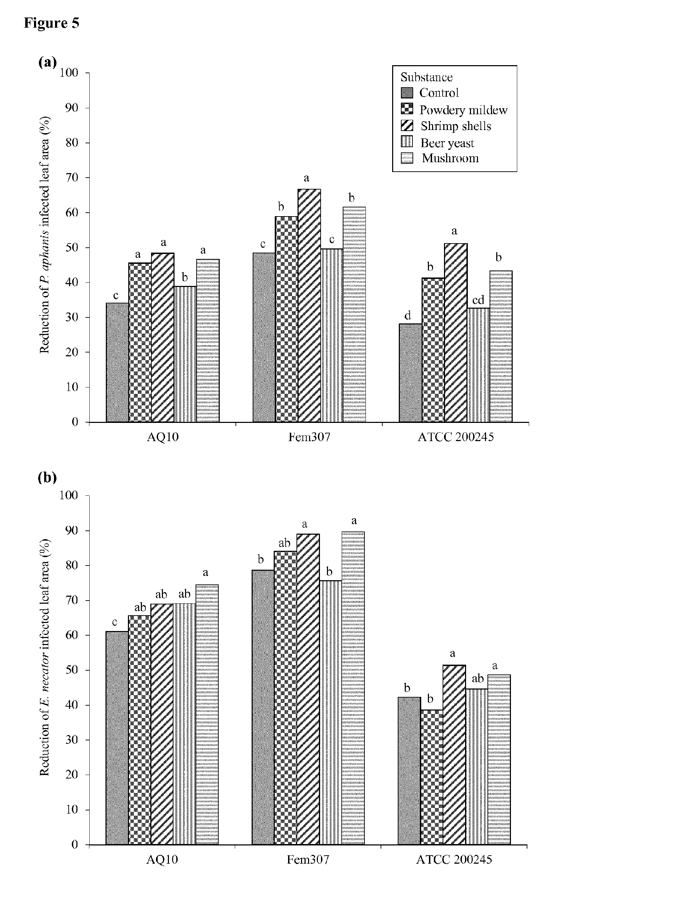

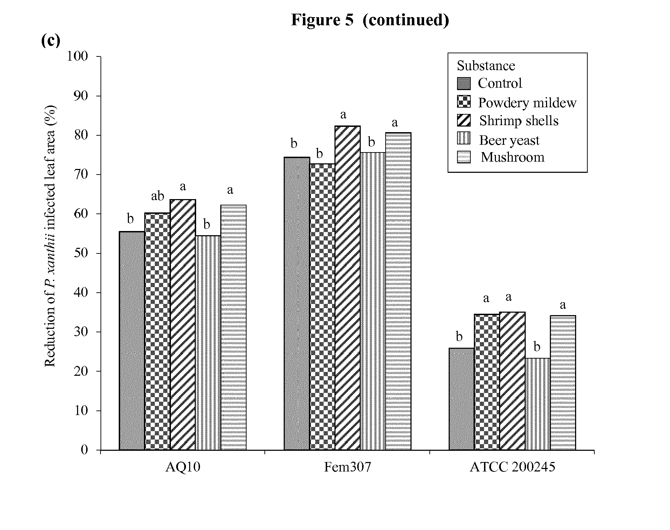

**Figure 5****:** This figure shows *P. xanthii, E. necator* and *P. aphanis* infected area on leaves sprayed with AQ10, Fem307 and ATCC 200245 stimulated by different substances (table 2). Untreated conidia were used as control. Columns with the same design represent the same *A. quisqualis* strain. Columns with the same letters (a-c) do not significantly differ (P ≤ 0.05, Tukey's test). Presented values are means of three independent experiments with three replicates per experiment

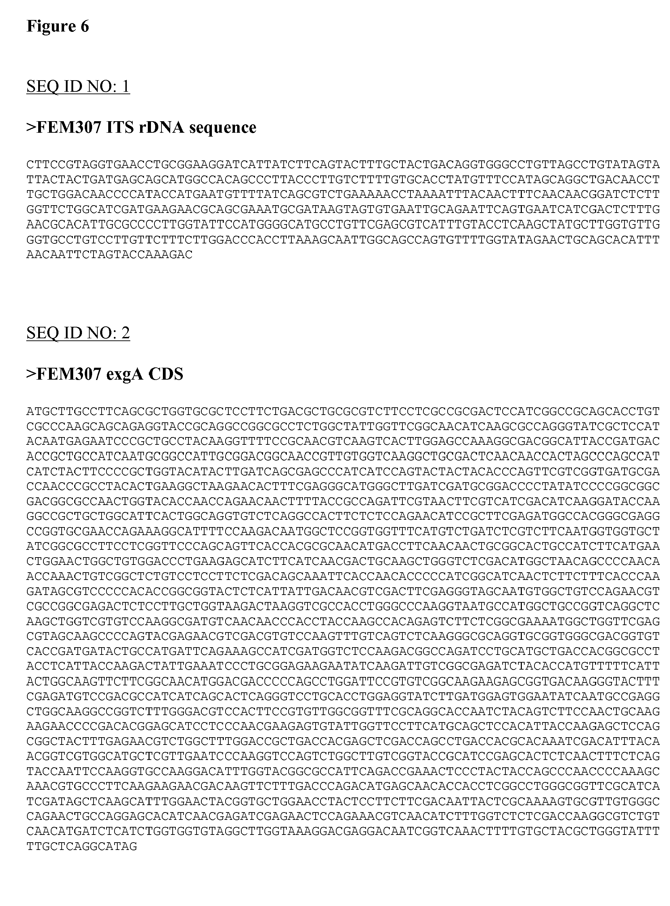

**Figure 6****:** This figure depicts a particular ITS rDNA sequence (SEQ ID NO: 1) and a particular exgA sequence (SEQ ID NO: 2) of the *Ampelomyces quisqualis* strain of the present invention as determined by sequencing the strain of deposit CBS 126895. ITS rDNA sequences are commonly used to identify fungal species. Strains within species are commonly identified using molecular markers, as a strain-specific sequence in a conserved area of DNA (e.g. a gene). SEQ ID NO: 1 comprises (in this order of sequences) a partial sequence of the 18S ribosomal RNA gene, internal transcribed spacer 1, the 5.8S ribosomal gene, and a partial sequence of internal transcribed spacer 2.

### SUMMARY OF THE INVENTION

The present invention, among others, relates to *(Ampelomyces quisqualis)* fungus, such as the novel fungal strain *Ampelomyces quisqualis* Fem307 and variants thereof.

The present invention also relates to fungal preparations, such as a preparation of the novel fungal strain *Ampelomyces quisqualis* Fem307 or of variants thereof.

The present invention also relates to uses and methods involving such novel fungi or fungal preparations for treating powdery mildews in plants.

The present invention also relates to uses or methods involving i) a mushroom preparation and/or ii) a shrimp shell preparation for treating powdery mildews with *Ampelomyces quisqualis.*

The invention further relates to the use of i) a mushroom preparation, particularly from *Agaricus bisporus,* and/or ii) a shrimp shell preparation, for enhancing biocontrol efficacy and/or fungicide efficacy of *Ampelomyces quisqualis.*

Preferably, and this is applicable to the whole application, the mushrooms are edible mushrooms, more preferably edible cultivated mushrooms.

Generally, the present invention predominantly relates to products, uses and methods as defined in the claims herein.

### DETAILED DESCRIPTION OF THE INVENTION

The present inventors have performed numerous studies and have succeeded in solving the above problems. Accordingly, the present invention, among others, discloses novel and unexpected ways of treating powdery mildew, as well as related subject-matter.

In a first aspect, the present invention relates to a fungal strain, more preferably an *Ampelomyces quisqualis* strain. Said strain according to the first aspect may be selected from the group consisting of i) *Ampelomyces quisqualis* Fem307, ii) a strain obtainable from deposit CBS 126895, iii) a strain comprising an ITS rDNA sequence that has at least 99.0 % pairwise identity to an ITS rDNA sequence of a strain as defined in item i) or ii) (preferably to an ITS rDNA sequence of SEQ ID NO: 1), and iv) a strain having an exgA gene that has at least 99.7 % pairwise identity to an exgA gene of a strain as defined in item i) or ii) (preferably to an exgA gene of SEQ ID NO: 2).

In said first aspect, the present invention likewise also relates to an *Ampelomyces quisqualis* fungus that may be selected from the group consisting of i) a fungus of *Ampelomyces quisqualis* Fem307, ii) a fungus obtainable from deposit CBS 126895, iii) a fungus comprising an ITS rDNA sequence that has at least 99.0 % pairwise identity to an ITS rDNA sequence of a fungus as defined in item i) or ii) (preferably to an ITS rDNA sequence of SEQ ID NO: 1), and iv) a fungus having an exgA gene that has at least 99.7 % pairwise identity to an exgA gene of a fungus as defined in item i) or ii) (preferably to an exgA gene of SEQ ID NO: 2).

Generally, the terms *"Ampelomyces quisqualis"* and *"A. quisqualis"* herein refer to the well-known anamorphic fungi of *Ampelomyces quisqualis. Ampelomyces quisqualis* is generally known as a hyperparasite of powdery mildew fungi, i.e. of plant pathogenic fungi causing powdery mildew(s). Preferably, an *A. quisqualis* in accordance with the invention is *Ampelomyces quisqualis* strain Fem307, a strain obtainable from deposit CBS 126895 or a variant thereof as defined herein. As used herein, a "strain of the invention" may also be referred to as a "fungus of the invention" or *"Ampelomyces quisqualis* of the invention".

In accordance with the present invention, *Ampelomyces quisqualis* strain Fem307, which was isolated from *Erysiphe necator* in Italy, has been deposited under the Budapest Treaty at the CBS (Centraalbureau voor Schimmelcultures) on May 6, 2010, and has been assigned accession number CBS 126895. *Ampelomyces quisqualis* FEM307 can be cultivated on several common microbiological media. A good example is Potato Dextrose Agar (PDA) (e.g. PDA of Oxoid or Difco). *A. quisqualis* FEM307 is a slow growing fungus. On PDA it produces many black pycnidia within one or two weeks. The fungus grows well under UV light and an optimal temperature range of 20-25°C. The pure culture of *A. quisqualis* FEM307 should be transferred regularly into a new, fresh medium, e. g. once every three to four weeks. On agar substrate, conidia which are produced by the micro-organism can be harvested and then seeded onto a new agar substrate. However, periodical transfer of conidia into new media may give rise to a mutagenic stress and in consequence a genetic drift of the culture may occur. Thus, such conservation of the culture is suitable mainly for short periods of time. For storage periods of up to about 6 months, *A. quisqualis* is put on PDA into tube and then it is kept at 4° C. Long term storage may also be achieved by freeze drying the conidia into vials at -80°C. Such freeze dried conidia preserve their viability over prolonged storage periods. As an alternative, Microbank® can be used for long term storage. The conditions for testing viability are given by the storage method (On PDA at 4°C growth of colonies (expected 100%). Freeze dried: growth of colonies after plating on PDA at 20-25°C. The viability is measured as percentage of CFU/conidia. Microbank®: viable colonies on PDA, 20-25°C).

There are no expected adverse effects on humans or the environment of *Ampelomyces quisqualis.* Based on required toxicity tests, no risks to humans are expected from handling or eating food containing small amounts of *A. quisqualis.* Applicators are instructed to use appropriate protective equipment, including a respirator to minimize their inhalation of dust or mist.

*A. quisqualis* is an environmentally friendly microbial biocontrol agent. It acts as a mycoparasite and production of toxins or antibiotics was never demonstrated. In Europe a strain of *A. quisqualis* is included in Annex 1 (Directive 414/92) and currently used worldwide as a biofungicide. When used as biofungicide, this fungus presents no known risks to plants, beneficial insects, or other non-target organisms. However, it is not allowed to be applied directly to water or be used in ways that could contaminate water.

A. quisqualis naturally occurs as hyperparasite of powdery mildews. It infects and forms pycnidia (fruiting bodies) within powdery mildew hyphae, conidiophores and cleistothecia. This parasitism reduces growth and may eventually kill the mildew colony. Parasitized mildew colonies are dull, flattened, and off-white to gray in colour and powdery mildews' conidia is reduced.

The pycnidia of A. quisqualis FEM307 vary in shape depending upon the organ in which they form. Within conidiophores they are pear-shaped, within hyphae they are spindle-shaped, and within cleistothecia they may be nearly spherical. Pycnidia contain fusiform or ellipsoidal conidia that are 11.5-14.5 X 2.5-3.5 micrometers. The conidia may be exuded when parasitized tissues or cultures on medium are exposed to free water or humidity near saturation.

Molecular analysis can be based on ITS sequencing. In particular the ITS region of the nrDNA of *A. quisqualis* FEM307 can be amplified using the specific fungal primers ITS1 and ITS4 with the following cycling parameters: initial denaturing step at 95°C for 3 min; 35 cycles of denaturing step at 95°C for 30 s, primer annealing at 60°C for 30 s, and extension step at 72°C for 30 s; and a final extension at 72°C for 7 min. PCR products are purified and sequenced. The sequences can be blasted and compared with deposited sequence (i.e. Genebank) of other *A. quisqualis* strains and related species.

The proposed taxonomic designation is *Ampelomyces quisqualis* FEM307. The organism is not genetically modified.

As used herein, a "strain obtainable from" said deposit also includes a "fungus obtainable from" said deposit and may also be referred to herein as an *"A. quisqualis* obtainable from" said deposit, wherein all of the above are included within an *Ampelomyces quisqualis* of the first aspect herein.

Also variants of *A. quisqualis* strain Fem307, as well as variants of an *A. quisqualis* obtainable from deposit CBS 126895, are explicitly included within the *Ampelomyces quisqualis* of the present invention. Such variants include but are not limited to mutants of *Ampelomyces quisqualis* strain Fem307 (particularly mutants having all of the identifying characteristics of *A. quisqualis* Fem307).

Such variants also include *A. quisqualis* fungi having an ITS rDNA sequence that has at least 98.5 % (preferably at least 99.0 %, such as at least 99.1, 99.2, 99.3, 99.4, 99.5, 99.6, 99.7, 99.8 or 99.9 %) pairwise identity to an ITS rDNA sequence of *A. quisqualis* Fem307, preferably to SEQ ID NO: 1. Such variants also include *A. quisqualis* fungi having an exgA gene that has at least 99.6 % (preferably at least 99.7, such as at least 99.75, 99.8, 99.85, 99.9 or 99.95 %) pairwise identity to an exgA gene of *A. quisqualis* Fem307, preferably to SEQ ID NO: 2. Pairwise identity in context with the present invention may readily be determined by the skilled person - particularly also in view of the teachings in the present examples. Accordingly, preferably herein, said reference "ITS rDNA sequence" refers to the sequence of SEQ ID NO: 1. Preferably herein, said reference "exgA gene" refers to the sequence of SEQ ID NO: 2.

In a second aspect, the present invention relates to a fungal preparation, more preferably an *Ampelomyces quisqualis* preparation. Said fungal preparation of the second aspect may be selected from the group consisting of i) a preparation of *Ampelomyces quisqualis* Fem307, ii) a preparation of a fungus obtainable from deposit CBS 126895, iii) a preparation of a fungus having an ITS rDNA that has at least 99.0 % pairwise identity to an ITS rDNA of a fungus as defined in item i) or ii) (preferably to an ITS rDNA sequence of SEQ ID NO: 1), and iv) a preparation of a fungus having an exgA gene that has at least 99.7 % pairwise identity to an exgA gene of a fungus as defined in item i) or ii) (preferably to an exgA gene of SEQ ID NO: 2). In certain preferred embodiments of items iii) and iv) of the second aspect correspond to those described herein in connection with the first aspect.

As used herein, a fungal preparation preferably refers to a preparation comprising fungi of the present invention. Accordingly, a fungal preparation of the present invention may also be a preparation comprising variants of fungi of the present invention, such as variants of *Ampelomyces quisqualis* strain Fem307, wherein said variants are as described hereinabove. Generally herein, a fungal preparation may e.g. be a wettable powder, a liquid, a dust, a spray, and granules.

Generally herein, the form of the fungi of the present invention (such as the fungi contained in a fungal preparation of the present invention) is not particularly limited. In certain preferred embodiments, the fungi of the invention are present in the form of spores. In certain preferred embodiments, the fungi of the invention are present in the form of conidia.

In certain embodiments, the fungal preparation is a liquid preparation. Accordingly, in certain embodiments, the fungal preparations herein are suspensions of spores or conidia of the respective *Ampelomyces quisqualis.* In other embodiments, the fungal preparation is a dry preparation, such as a dry spore preparation. In some embodiments, the fungal preparation is dry-formulated in granules, preferably in stable, water-dispersible granules.

The fungal preparations herein may preferably comprise additives. Non-limiting examples of such additives include one or more additives selected from the group consisting of mineral oils, milk proteins and maltodextrins. In certain embodiments, the fungal preparations herein comprise mineral oils, such as a mineral oil-based wetting agent. In certain embodiments, the fungal preparations herein are administered in conjunction with mineral oils, such as with a mineral oil-based wetting agent. The skilled person will readily be able to determine suitable concrete such formulations, non-limiting examples of which include an emulsion of 0.3-1% mineral oil, such as paraffin oil. In certain embodiments, the fungal preparations herein comprise milk proteins. In certain embodiments, the fungal preparations herein comprise maltodextrins.

In certain embodiments herein, the fungal preparations herein additionally comprise or are administered in conjunction with one or more additional fungicides, preferably selected from the group consisting of triforine, quinomethionate, triadimefon, myclobutanil and other fungicides suitable for treating powdery mildews.

In certain embodiments herein, the fungal preparations herein additionally comprise or are administered in conjunction with one or more acaricides.

In certain embodiments herein, the fungal preparations herein additionally comprise or are administered in conjunction with one or more insecticides.

In certain embodiments, said fungal preparation may also be referred to as a biofungicide. Preferably herein, said biofungicide is a biofungicide against powdery mildews in plants, which may also be referred to herein as a biofungicide for treating powdery mildews in plants. Accordingly, the present invention also relates to biofungicides comprising fungi of the invention. Accordingly, the present invention also relates to biofungicides comprising a fungal preparation of the invention

In a third aspect, the present invention relates to the use of fungi of the invention, such as of a fungal strain of the invention, or of a fungal preparation of the invention for treating powdery mildews, preferably for treating powdery mildews in plants.

Powdery mildew is a well-known fungal disease that affects a wide variety of plants. Since it may be caused by various different fungi (particularly in the order *Erysiphales*), powdery mildew strictly speaking occurs in certain distinct forms. Hence, it may exchangeably also be referred to as "powdery mildews" herein. In each case, it preferably refers to the entirety of powdery mildew diseases.

Accordingly, powdery mildew as referred to herein is not particularly limited and includes all powdery mildews known to the skilled person. Non-limiting examples thereof include, but are not limited to powdery mildew of cucumber *(Podosphaera xanthii),* strawberry (*Podosphaera aphanis),* grapevine *(Erysiphe necator),* apple *(Podosphaera leucotricha),* onions *(Leveillula taurica),* cereals *(Blumeria graminis),* endive, lettuce *(Erysiphe cichoracearum),* brassicaceae *(Erysiphe cruciferarum),* tomato *(Erysiphe lycopersici),* carrots *(Erysiphe heraclei),* beets *(Erysiphe polygoni).* Particularly preferred powdery mildews herein are powdery mildew of cereals *(Blumeria graminis),* cucumber *(Podosphaera xanthii),* strawberry *(Podosphaera aphanis)* and grapevine *(Erysiphe necator).* Accordingly, in preferred embodiments herein, powdery mildews are selected from the group consisting of powdery mildews of cucumber, strawberry, grapevine, apple, onions, cereals, endive, lettuce, brassicaceae, tomato, carrots, and beets, more preferably from cereals, cucumber, strawberry and grapevine. A preferred non-limiting example of cereals is wheat. Likewise, in preferred embodiments herein, powdery mildews are powdery mildew caused by fungi selected from the group consisting of *Podosphaera xanthii, Podosphaera aphanis, Erysiphe necator, Podosphaera leucotricha, Leveillula taurica, Blumeria graminis, Erysiphe cichoracearum, Erysiphe cruciferarum, Erysiphe lycopersici, Erysiphe heraclei,* and *Erysiphe polygoni,* more preferably by fungi selected from the group consisting of *Blumeria graminis, Podosphaera xanthii, Podosphaera aphanis,* and *Erysiphe necator.* In certain preferred embodiments herein powdery mildews are selected from those caused by the genera *Podosphaera* and *Erysiphe.* Another preferred genus is *Blumeria.*

As used herein, the term "for treating powdery mildew in plants" and suchlike terms are equivalently used with the term "for treating plants against powdery mildews". This term preferably also includes the term "for treating plants affected by powdery mildews".

In any case, it will be appreciated by the skilled person that the fungi and fungal strains of the invention and fungal preparations of the invention may be used against powdery mildew fungi or powdery mildew diseases, respectively, which diseases affect plants (and may e.g. be prevented or treated in context with the present invention). Likewise, it will be appreciated by the skilled person that the fungi and fungal preparations of the present invention may be used as a fungicide, particularly as a fungicide against powdery mildew fungi [or in other words a fungicide against powdery mildew(s) or in other words a fungicide to treat powdery mildew(s)].

In a fourth aspect, the present invention relates to a method of treating powdery mildews, preferably for treating powdery mildews in plants, wherein said method preferably comprises a step of contacting the plant(s) to be treated with a fungus of the invention (such as with a fungal strain of the invention) or with a fungal preparation of the invention.

In preferred embodiments of the third and fourth aspects, the use or method, respectively, additionally comprises adding a mushroom preparation, particularly from *Agaricus bisporus.* In preferred embodiments of the third and fourth aspects, the use or method, respectively, additionally comprises adding a shrimp shell preparation. In preferred embodiments of the third and fourth aspects, the use or method, respectively, additionally comprises adding i) a mushroom preparation, particularly from *Agaricus bisporus,* and ii) a shrimp shell preparation. Accordingly, in preferred embodiments of the third and fourth aspects, the use or method, respectively, additionally comprises adding i) a mushroom preparation, particularly from *Agaricus bisporus,* and/or ii) a shrimp shell preparation.

Embodiments of the mushroom preparation correspond to embodiments described elsewhere herein. Embodiments of the shrimp shell preparation correspond to embodiments described elsewhere herein.

In preferred embodiments of the third and fourth aspects, said fungal strain or fungal preparation, respectively, has improved biocontrol efficacy as compared to a strain or preparation, respectively of *Ampelomyces quisqualis* AQ10. In preferred embodiments of the third and fourth aspects, said fungal strain or fungal preparation, respectively, has improved fungicide efficacy as compared to a strain or preparation, respectively of *Ampelomyces quisqualis* AQ10. In preferred embodiments of the third and fourth aspects, said fungal strain or fungal preparation, respectively, has improved biocontrol efficacy and fungicide efficacy as compared to a strain or preparation, respectively of *Ampelomyces quisqualis* AQ10. In certain embodiments biocontrol efficacy and/or fungicide efficacy is/are additionally improved by adding said mushroom preparation and/or shrimp shell preparation as described herein. Such enhancement of biocontrol efficacy may be the consequence of an improvement of germination.

As used herein "improved fungicide efficacy" means that there is an improved efficacy in the inhibition of plant pathogenic fungi or fungal spores, particularly of powdery mildew mycelium or their spores. Such improved inhibition includes, but is not limited to, a decreased growth of said pathogenic fungi, mycelium and/ or production of their spores. As used herein "improved biocontrol efficacy" means that there is an improved overall efficacy in biocontrol, such as in context with a biocontrol application in plants. Such application e.g. includes administration of fungi or fungal preparations of the invention to a plant or a group of plants affected by powdery mildew.

In a fifth aspect, the present invention relates to the use of a mushroom preparation, and/or ii) a shrimp shell preparation, in treating powdery mildews in plants with *Ampelomyces quisqualis.* Accordingly, in some embodiments, this aspect relates to use of a mushroom preparation in treating powdery mildews in plants with *Ampelomyces quisqualis.* In other embodiments, this aspect relates to use of a shrimp shell preparation, in treating powdery mildews in plants with *Ampelomyces quisqualis.* In further embodiments, this aspect relates to use of a mushroom preparation, and ii) a shrimp shell preparation, in treating powdery mildews in plants with *Ampelomyces quisqualis.*

In a sixth aspect, the present invention relates to a method of treating powdery mildews in plants with *Ampelomyces quisqualis.* Said method preferably comprises a step of adding i) a mushroom preparation, and/or ii) a shrimp shell preparation. Accordingly, in some embodiments, the method comprises a step of adding a mushroom preparation. In other embodiments, the method comprises a step of adding a shrimp shell preparation. In other embodiments, the method comprises a step of adding i) a mushroom preparation, and ii) a shrimp shell preparation. Said preparation(s) is/are preferably added to at least one plant having powdery mildew (which may also be referred to herein as at least one plant affected by powdery mildew).

As used herein, a "mushroom" refers to real fungi. Preferably, a mushroom herein is a fungus having a stem, a cap and gills (i.e. lamellae) or pores on the underside of the cap. Preferably, a mushroom herein is a mushroom of the genus *Agaricus.* More preferably a mushroom herein is an *Agaricus bisporus* or *Agaricus campestris,* preferably *Agaricus bisporus.* In certain embodiments herein, the mushroom is not *Coprinus comatus.*

As used herein, "shrimp shells" are not particularly limited. Generally, shrimps are well-known crustaceans found widely around the world in both fresh and salt water, whereas the term shrimp shells refers to the exoskeletons thereof.

Generally herein, a mushroom preparation is intended to refer to a preparation comprising mushrooms or mushroom elements. Preferably a mushroom preparation is prepared from whole mushrooms. Alternatively, a mushroom preparation is prepared from mushroom pieces. A mushroom preparation herein may generally be in liquid or solid form. A preferred liquid form is a suspension, most preferably a suspension of a powder of mushrooms.

In preferred embodiments, a mushroom preparation is prepared by grinding mushrooms or mushroom pieces. Grinding may be performed in a liquid. Exemplary liquids include water and saline. The liquid may or may not be buffered. The result of grinding may be referred to herein as ground mushroom or ground mushroom pieces. Grinding herein is not particularly limited. Various ways of grinding are known to the skilled person. In some embodiments, the mushrooms or mushroom pieces are dry or dried, respectively, before grinding them. After performing grinding in solution, the ground preparation is preferably dried.

Accordingly, a mushroom preparation herein may be a ground preparation. Optionally said preparation is resuspended in a suitable liquid to obtain a mushroom preparation in the form of a suspension. Exemplary liquids include water and saline. The liquid may or may not be buffered.

In preferred embodiments of the mushroom preparation, mushroom or mushroom pieces, respectively, and preferably the ground preparation, are subject to mechanically disruption (which may also be referred to herein as lysis, braking up, or the like). Lysis may be performed in solution, such as in a lysis solution. Exemplary lysis solutions include water and saline. The lysis solution may or may not be buffered. The result of lysing may be referred to herein as a mushroom powder or fine powder, respectively. Mechanical disruption herein is not particularly limited. Various ways of disruption (including the use of a tissue lyser) are known to the skilled person. In some embodiments, the (preferably ground) mushrooms or mushroom pieces are dry or dried, respectively, before mechanically disrupting them. After performing lysis in solution, the lysis preparation is preferably dried to obtain a powder, preferably a fine powder.

Accordingly, a mushroom preparation herein may be in the form of a powder, preferably a fine powder. The powder may be obtained as described herein by a method involving grinding and/or mechanical disruption. Optionally said powder is resuspended in a suitable liquid to obtain a mushroom preparation in the form of a suspension. Exemplary liquids include water and saline. The liquid may or may not be buffered. Accordingly, in some embodiments, the mushroom preparation is a suspension of a ground and/or lysed mushroom preparation. Preferably, the mushroom preparation is a suspension of a mushroom powder. A preferred concentration of such suspension is 1 g per liter.

In preferred embodiments, the mushroom preparation, preferably the suspension, preferably the suspension of a mushroom powder, is subjected to a heat treatment step. An exemplary not limiting heat treatment comprises exposure to 100-140 °C, such as 110-130 °C, such as about 120°C, for a time of from 10 to 30 minutes, preferably 15 to 25 minutes, preferably for about 20 minutes. Heat treatment may e.g. be achieved by means of an autoclave. Suitable parameters for heat treatment such as temperature and duration will readily be apparent to the skilled person and may e.g. be suitable to disrupt agglomerates. Moreover, the present inventors have surprisingly found that heat treatment further improves the beneficial effects of the mushroom preparation.

In preferred embodiments, the mushroom preparation, preferably the suspension, preferably the suspension of a mushroom powder, is subjected to one or more filtering steps. Suitable parameters for filtering such as filter sizes will readily be apparent to the skilled person and may e.g. be desirable to homogenize the mushroom preparation.

In accordance with the present invention, the mushroom preparation may be obtainable by any of the methods described herein.

Generally herein, a shrimp shell preparation is intended to refer to a preparation comprising shrimp shells or shrimp shell elements. Preferably a shrimp shell preparation is prepared from whole shrimp shells. Alternatively, a shrimp shell preparation is prepared from shrimp shell pieces. A shrimp shell preparation herein may generally be in liquid or solid form. A preferred liquid form is a suspension, most preferably a suspension of a shrimp shell powder.

In preferred embodiments, a shrimp shell preparation is prepared by grinding shrimp shells or shrimp shell pieces. Grinding may be performed in a liquid. Exemplary liquids include water and saline. The liquid may or may not be buffered. The result of grinding may be referred to herein as ground shrimp shell or ground shrimp shell pieces. Grinding herein is not particularly limited. Various ways of grinding are known to the skilled person. In some embodiments, the shrimp shells or shrimp shell pieces are dry or dried, respectively, before grinding them. After performing grinding in solution, the ground preparation is preferably dried.

Accordingly, a shrimp shell preparation herein may be a ground preparation. Optionally said preparation is resuspended in a suitable liquid to obtain a shrimp shell preparation in the form of a suspension. Exemplary liquids include water and saline. The liquid may or may not be buffered.

In preferred embodiments of the shrimp shell preparation, shrimp shell or shrimp shell pieces, respectively, and preferably the ground preparation, are subject to mechanically disruption (which may also be referred to herein as lysis, braking up, or the like). Lysis may be performed in solution, such as in a lysis solution. Exemplary lysis solutions include water and saline. The lysis solution may or may not be buffered. The result of lysing may be referred to herein as a shrimp shell powder or fine powder, respectively. Mechanical disruption herein is not particularly limited. Various ways of disruption (including the use of a tissue lyser) are known to the skilled person. In some embodiments, the (preferably ground) shrimp shells or shrimp shell pieces are dry or dried, respectively, before mechanically disrupting them. After performing lysis in solution, the lysis preparation is preferably dried to obtain a powder, preferably a fine powder.

Accordingly, a shrimp shell preparation herein may be in the form of a powder, preferably a fine powder. The powder may be obtained as described herein by a method involving grinding and/or mechanical disruption. Optionally said powder is resuspended in a suitable liquid to obtain a shrimp shell preparation in the form of a suspension. Exemplary liquids include water and saline. The liquid may or may not be buffered. Accordingly, in some embodiments, the shrimp shell preparation is a suspension of a ground and/or lysed shrimp shell preparation. Preferably, the shrimp shell preparation is a suspension of a shrimp shell powder. A preferred concentration of such suspension is 1 g per liter.

In preferred embodiments, the shrimp shell preparation, preferably the suspension, preferably the suspension of a shrimp shell powder, is subjected to a heat treatment step. An exemplary not limiting heat treatment comprises exposure to 100-140 °C, such as 110-130 °C, such as about 120°C, for a time of from 10 to 30 minutes, preferably 15 to 25 minutes, preferably for about 20 minutes. Heat treatment may e.g. be achieved by means of an autoclave. Suitable parameters for heat treatment such as temperature and duration will readily be apparent to the skilled person and may e.g. be suitable to disrupt agglomerates. Moreover, the present inventors have surprisingly found that heat treatment further improves the beneficial effects of the shrimp shell preparation.

In preferred embodiments, the shrimp shell preparation, preferably the suspension, preferably the suspension of a shrimp shell powder, is subjected to one or more filtering steps. Suitable parameters for filtering such as filter sizes will readily be apparent to the skilled person and may e.g. be desirable to homogenize the shrimp shell preparation.

In accordance with the present invention, the shrimp shell preparation may be obtainable by any of the methods described herein.

Generally herein, a mushroom preparation may e.g. be a wettable powder, a liquid, a dust, a spray, and granules. Generally herein, a shrimp shell preparation may e.g. be a wettable powder, a liquid, a dust, a spray, and granules. Preferably, the liquid preparation is a suspension.

Preferred non limiting examples of obtaining such preparations are described herein. In any case, is envisaged that the skilled reader will readily be in the position to prepare such preparations in alternative ways based on his skills and the disclosures herein.

In preferred embodiments herein, the mushroom preparation is a suspension of a mushroom powder, particularly from *Agaricus bisporus.* Likewise, in preferred embodiments herein, the shrimp shell preparation is a suspension of a shrimp shell powder. As used herein, a "mushroom powder" may also be referred to as a "powder derivable from a mushroom". As used herein, a "shrimp shell powder" may also be referred to as a "powder derivable from shrimp shells".

Accordingly, in preferred embodiments herein, the mushroom preparation is a suspension of a powder derivable from an mushroom, particularly from *Agaricus bisporus.* Likewise, in preferred embodiments herein, the shrimp shell preparation is a suspension of a powder derivable from shrimp shells. In other preferred embodiments herein, the mushroom preparation is a powder derivable from a mushroom, particularly from *Agaricus bisporus.* Likewise, in preferred embodiments herein, the shrimp shell preparation is a powder derivable from shrimp shells. Such powders may be obtained by mechanically disrupting mushrooms or shrimp shells, respectively, optionally while being suspended in a suitable solvent. A suitable solvent is considered to be readily determined by the skilled person and may for example be distilled water or a buffered solution. The solvent may also be a saline solution such as a water saline solution. Certain preferred ways of how to obtain a powder derivable from mushrooms are described elsewhere herein. Certain preferred ways of how to obtain a powder derivable from shrimp shells are described elsewhere herein.

In preferred embodiments herein, the mushroom preparation is from *Agaricus bisporus.* Accordingly, in preferred embodiments, the mushroom preparation is an *Agaricus bisporus* preparation. In certain embodiments herein, the mushroom preparation used in context with the invention is not a *Coprinus comatus* preparation.

In preferred embodiments of the fifth and sixth aspects, said *Ampelomyces quisqualis* is a fungus or fungal strain according to the first aspect or is or comprises a fungal preparation according to the second aspect of the invention. Embodiments of said fungus or fungal strain correspond to the embodiments described in context with said first aspect. Embodiments of said fungal preparation correspond to the embodiments described in context with said second aspect.

Generally herein, embodiments described in connection with the third and fourth aspect of the invention are also contemplated as embodiments of the fifth and sixth aspect of the invention, and vice versa. Generally herein, suchlike embodiments include analogous embodiments.

In other preferred embodiments of the fifth and sixth aspects, said *Ampelomyces quisqualis* is any *Ampelomyces quisqualis* or *Ampelomyces quisqualis* preparation effective in treating powdery mildew in plants. Preferably, said *Ampelomyces quisqualis* is AQ10 or an AQ10 preparation. In other embodiments, said *Ampelomyces quisqualis* is *Ampelomyces quisqualis* ATCC 200245 or an ATCC 200245 preparation.

In preferred embodiments herein, the mushroom preparation and/or shrimp shell preparation i) improve(s) germination of *Ampelomyces quisqualis* conidia, and/or ii) enhance(s) biocontrol efficacy of the *Ampelomyces quisqualis,* and/or iii) enhance(s) fungicide efficacy of the *Ampelomyces quisqualis.* That is, in exemplary embodiments, the mushroom preparation and/or shrimp shell preparation improve(s) germination of *Ampelomyces quisqualis* conidia. In exemplary embodiments, the mushroom preparation and/or shrimp shell preparation enhance(s) biocontrol efficacy of the *Ampelomyces quisqualis.* In exemplary embodiments, the mushroom preparation and/or shrimp shell preparation enhance(s) fungicide efficacy of the *Ampelomyces quisqualis.* In certain preferred embodiments, the mushroom preparation and/or shrimp shell preparation improve(s) germination of *Ampelomyces quisqualis* conidia and enhance(s) biocontrol efficacy of the *Ampelomyces quisqualis.* Enhancement of biocontrol efficacy of the *Ampelomyces quisqualis* may be the consequence of the improvement of germination. In other words, said improvement of germination may cause said enhancement of biocontrol efficacy. In exemplary embodiments, the mushroom preparation and/or shrimp shell preparation additionally enhance(s) fungicide efficacy of the *Ampelomyces quisqualis.*

As used herein "enhancing of fungicide efficacy" means that an improved efficacy in the inhibition of plant pathogenic fungi or fungal spores, particularly of powdery mildew mycelium or their spores, can be achieved. Such improved inhibition includes, but is not limited to, a decreased growth of said plant pathogenic fungi, mycelium and/or production of their spores. As used herein "enhancing biocontrol efficacy" means that an improved overall efficacy in biocontrol can be achieved, such as in context with a biocontrol application in plants. Such application e.g. includes administration of *Ampelomyces quisqualis* or *Ampelomyces quisqualis* preparations of the invention to a plant or a group of plants affected by powdery mildew.

Generally herein, in certain preferred embodiments, *Ampelomyces quisqualis* is used in the form of conidia. Accordingly, as exemplary but not limiting embodiments herein, powdery mildews are treated or are to be treated with a suspension of *Ampelomyces quisqualis* conidia. Accordingly, a fungal preparation of the present invention may be a suspension of *Ampelomyces quisqualis* conidia. Such suspensions may e.g. comprise from 1 × 10⁵ conidia ml⁻¹ to 1 × 10⁷ conidia ml⁻¹, such as from 1 × 10⁵ conidia ml⁻¹ to 5 × 10⁶ conidia ml⁻¹, such as from 5 × 10⁵ conidia ml⁻¹ to 1 × 10⁶ conidia ml⁻¹.

In an even further aspect, the invention relates to the use of i) a mushroom preparation, and/or ii) a shrimp shell preparation, for enhancing biocontrol efficacy and/or fungicide efficacy of *Ampelomyces quisqualis.* In some embodiments, the invention relates to the use of a mushroom preparation for enhancing fungicide efficacy of *Ampelomyces quisqualis.* In some embodiments, the invention relates to the use of a shrimp shell preparation for enhancing fungicide efficacy of *Ampelomyces quisqualis.* In other embodiments, the invention relates to the use of a mushroom preparation for enhancing fungicide efficacy and biocontrol efficacy of *Ampelomyces quisqualis.* In other embodiments, the invention relates to the use of a shrimp shell preparation for enhancing biocontrol efficacy and fungicide efficacy of *Ampelomyces quisqualis.* In even further embodiments, the invention relates to the use of i) a mushroom preparation, and ii) a shrimp shell preparation, for enhancing biocontrol efficacy and/or fungicide efficacy of *Ampelomyces quisqualis.*

Also in preferred embodiments of the latter aspect and other aspects of the invention, the mushroom preparation is from *Agaricus bisporus.* Accordingly, in preferred embodiments, the mushroom preparation is an *Agaricus bisporus* preparation. Generally, embodiments of the mushroom and/or shrimp shell preparation used in any of the respective aspects include all embodiments thereof described herein. Likewise, embodiments of said *Ampelomyces quisqualis* of the latter aspect correspond to embodiments described herein.

In a further aspect, the invention relates to a biofungicide comprising a) an activator of *Ampelomyces quisqualis,* selected from a mushroom preparation, a shrimp shell preparation, and a mixture thereof and b) a fungal strain or a fungal preparation of *Ampelomyces quisqualis,* in particular the fungal strain or fungal preparation according to claims 1 and 2.

Generally herein, as non-limiting examples, the uses and methods disclosed herein may be for plants growing in the open field, greenhouse or in individual settings such as tissue-culture settings. Accordingly, as non-limiting examples, the *Ampelomyces quisqualis* fungi or fungal preparations herein may be applied to plants growing in the open field, greenhouse or in individual settings such as tissue-culture settings. Preferred embodiments comprise open field applications. Other preferred embodiments comprise greenhouse applications. Other preferred embodiments comprise tissue-culture applications.

Generally, the handling of *Ampelomyces quisqualis* (including, but not limited to culturing, storage, fermentation, formulation and application) is well-known to the skilled person, particularly in view of the numerous publications on AQ10 including e.g. US patent publication US5190754 and the other literature herein, all of which are incorporated by reference herein. The same applies in view of the teachings of the Examples disclosed herein below. Accordingly, it is e.g. envisaged that suitable concentrations of *Ampelomyces quisqualis* for application to plants can easily be determined by the skilled person.

As non-limiting example, the *Ampelomyces quisqualis* fungi or fungal preparations herein may be applied by ground spraying. Exemplary application frequencies herein are from 2 to 12 applications per season, such as 2, 3, 4, 5, 6, 7, 8, 9, 10, 11 or 12 applications per season. Preferred numbers of application can easily be determined by the skilled person and e.g. depend on disease pressure.

As non-limiting example, the *Ampelomyces quisqualis* fungi or fungal preparations herein may be applied in doses of about 2 x 10¹¹ spores/ha, such as from 1 × 10¹¹ to 5 × 10¹¹, 1.5 × 10¹¹ to 3 × 10¹¹, 1.6 × 10¹¹ to 2 × 10¹¹, or 1.7 × 10¹¹ to 1.9 × 10¹¹ spores/ha.

As non-limiting examples, the *Ampelomyces quisqualis* fungi or fungal preparations herein may be applied to the shoot, stem, leaves, seeds, and/or vegetative propagules of one or more plants, preferably to the shoot, stem, leaves and/or vegetative propagules of one or more plants, more preferably to the stem and/or leaves, preferably to the leaves of at least one plant.

Generally, in preferred embodiments of the uses and methods herein the fungi or fungal preparations herein are used in an amount effective against powdery mildew. An effective amount herein means an amount effective to achieve a significant inhibition of powdery mildews in at least one plant, particularly when compared to a control in the absence of said fungi or fungal preparation.

In preferred embodiments herein, the *Ampelomyces quisqualis* fungi or fungal preparation of the invention have fungicide efficacy and/or biocontrol efficacy, preferably fungicide efficacy and/or biocontrol efficacy as defined hereinabove.

In preferred embodiments herein, the *Ampelomyces quisqualis* fungi or fungal preparation of the invention are applied to plants that show symptoms of powdery mildew, such as at least one plant that show visible signs of powdery mildew. Such symptoms and signs are well-known to the skilled person and may include white powdery spots on leaves and/or stems. Accordingly, in preferred embodiments, the uses and methods disclosed herein are for treating plants that show symptoms of powdery mildew, such as visible signs of powdery mildew. Such symptoms and signs may include white powdery spots on leaves and/or stems.

The present invention refers in another aspect to the use of i) a mushroom preparation, particularly from *Agaricus bisporus,* and/or ii) a shrimp shell preparation, for enhancing biocontrol efficacy and/or fungicide efficacy of *Ampelomyces quisqualis,* particularly wherein said mushroom preparation and/or shrimp shell preparation and/or said *Ampelomyces quisqualis* is further defined as in any of the enclosed claims.

In preferred embodiments herein, none of the methods and uses are or involve a method for the treatment of the human or animal body by surgery or therapy. In preferred embodiments herein, none of the methods and uses herein are or involve a diagnostic method practiced on the human or animal body.

### EXAMPLES

The following examples are meant to further illustrate, but not limit, the invention. The examples comprise technical features, and it will be appreciated that the invention also relates to any combinations of the technical features presented in this exemplifying section.

### Example 1: Identification of the novel strain of Ampelomyces quisqualis Fem307

*A. quisqualis* Fem307 was grown on PDB + chloramphenicol at 150 rpm for 7 days at 25°C. Genomic DNA was extracted from fresh mycelial mats (approximately 150-200 mg) harvested by centrifugation using the Nucleo Spin Plant Kit (Macherey-Nagel, Germany). For the molecular characterization of *A. quisqualis* two genetic loci was amplified and sequenced. The ITS region of the nuclear ribosomal DNA was amplified using the primer ITS 1 and ITS4 (Table1) (Gardes and Bruns 1993). The complete coding domain sequence (CDS) of the exo-β-1,3-Glucanace gene (*exg*A) (Rotem et all. 1999) was also amplified and sequenced with newly designed primer sets (Table 1) and applying the strategy presented in Figure 1. PCR amplifications were performed in 20 µl reactions containing 1x DreamTaq reaction buffer supplemented with 2 mM MgCl₂ (Fermentas), 200 µl dNTPs, 0.5 µM each primer, 0.5 units DreamTaq polymerase (Fermentas) and 1 µl of template DNA. PCR reactions were run on a PTC-100 thermal cycler (MJ Research Inc) with the following cycling conditions: an initial step at 94°C for 5 min, followed by 35 cycles of 94°C for 30 sec, a primer dependent annealing temperature for 30 sec (Table 1) and 72°C for 3 min, and a final extension at 72°C for 10 min. PCR products were purified with EuroGOLD Cycle-Pure Kit (EuroClone) and a dye terminator-labelled cycle sequencing reaction was conducted with the BigDye Terminator v3.1 Cycle Sequencing Kit (Applied Biosystems). Reaction products were analyzed using an ABI 3130 Genetic Analyzer (Applied Biosystems).

The obtained sequence trace files were analyzed using the respective components of the DNAStar 5.0 software package (Lasergene Inc.). Multiple nucleotide sequence alignments were performed with MUSCLE (Edgar 2004). In the case of *exg*A gene multiple protein sequence alignment was performed and back-translated to nucleotide sequences with MUSCLE. Alignments were finally confirmed by visual inspection. Phylogenetic relationships between different strains of *A. quisqualis* were inferred by maximum-likelihood (ML) method. Prior to ML analysis, DNA substitution model parameters were estimated using Modeltest 3.7 (Posada and Crandall 1998) and the Akaike information criterion (AIC). For the ITS rDNA and *exg*A sequence data the submodels K2+I and TN93+G was selected respectively. ML heuristic searches were performed using 100 random taxon addition replicates with tree bisection and reconnection (TBR) branch swapping. Bootstrap support was inferred using 100 bootstrap replicates. Searches were performed with PAUP version 4.0b10 (Swofford 2002).

**Table 1: Primers used for amplifying and sequencing ITS rDNA and exgA loci in A. quisqualis (sequences: SEQ ID NOs: 3-10, from top to bottom)**

| Loci | Primer name | Primer seq | Tann | Ref. |
|---|---|---|---|---|
| ITS | ITS1 | TCCGTAGGTGAACCTGCGG | 54°C | Gardes and |
| | ITS4 | TCCTCCGCTTATTGATATGC | | Bruns 1993 |
| *exg*A | exgA-F2 | CCGCAACTCAAGCATTCCAACAAA | 58°C | |
| | exgA-R2 | TGCTAATAAAAGGCCCTCTCATCA | | |
| | exgA-994f | TCGGCGCCTTCCTCGGATCT | * | This study |
| | exgA-1131r | CGAGACCCAGCTTGCAGTCGT | * | |
| | exgA-1736f | GGACGACCCCCAGCCTGGAT | * | |
| | exgA-1883r | GGCCTTGCCAGCCTCAGCAT | * | |

| | | | | |
|---|---|---|---|---|
| * Internal primers used for sequencing exgA fragment. | | | | |

The phylogenetic relationships of nine selected strains of *A. quisqualis* reconstructed based on the ITS rDNA sequence and the *exg*A gene and presented in Figure 2. Both genetic loci support the grouping of *A. quisqualis* strains as e.g. described by Park et al. 2010. The nine strains used for the analysis belong to four major clades (1, 2, 3 and 5). Strain Fem307 is positioned in the 5^{th} clade while the commercially available *A. quisqualis* strain AQ10 is positioned in the 2^{nd} clade. Based on the ITS sequence the two strains share ∼85% pairwise identities (411 identical sites out of 486 aligned sites) while according to the *exg*A sequence the two strains share 95.5% pairwise identities (2,230 identical sites out of 2,334 aligned sites).

### Example 2: Comparison between A. quisqualis Fem307 and A. quisqualis AQ10

Cucumber *(Podosphaera xanthii),* strawberry *(Podosphaera aphanis)* and grapevine (*Erysiphe necator)* powdery mildew were used to evaluate the effectiveness of mycoparasitic strains of *A. quisqualis.* Fungal host *P. xanthii, P. aphanis and E. necator* were from populations collected in Trentino Alto Adige region between 2010 and 2011 on zucchini, strawberry and grapevine plants, respectively. Fresh colonies of powdery mildew were maintained in greenhouse controlled conditions at 25 ± 1°C and 70 ± 10 % of relative humidity (RH) and natural photoperiod. Subsequent inoculations of powdery mildew were carried out every 30 days by shaking leaves with sporulating mildew on 3-week-old plants having at least five true leaves.

The isolate of the present invention, Fem307, isolated from *Erysiphe necator* in Italy, was compared to the commercially available strain of *A. quisqualis* (AQ10 strain, AF035783, isolated from powdery mildew of *Chata edulis)* in Israel in terms of biocontrol against different powdery mildews *(P. xanthii, P. aphanis, E. necator).* AQ10 was obtained from the commercial company Intrachem bio Italia. Cultures were grown and maintained on potato dextrose agar (PDA, Oxoid, United Kingdom) solid medium at 25°C in the dark. For long-term storage, the strains were preserved at -80°C using microbank vials.

The ability of the two *A. quisqualis* strains to inhibit the sporulation of *P. xanthii, E. necator* and *P. aphanis* on their respective host plants has been evaluated under controlled conditions. Two-week old cucumber, grapevine and strawberry plants were inoculated with the respective powdery mildew by spraying a suspension of distilled water and Tween 80 (0.01%) containing 1 × 10⁵ conidia ml⁻¹ in case of *P. xanthii* or as a dry inoculation by shaking some infected leaves with sporulating mildew above the plants in case of *E. necator* and *P. aphanis.* For strawberry and grapevine powdery mildew the amount of inoculum was expressed as number of conidia counted by collecting them on glass slides placed between the plants during dry inoculation. The choice of different inoculation methods was based on preliminary trials carried out to optimize the methodology (data not shown). Powdery mildew sporulation occurred on upper leaf surface at different times: five, seven and twelve days after infection of cucumber, grapevine and strawberry plant, respectively and plants were immediately used in the bioassay. Powdery mildew sporulation appeared as a homogenous infection on cucumber and grapevine leaves, while several powdery spots appeared on strawberry leaves.

*A. quisqualis* strains conidia suspensions were prepared as follows. *A. quisqualis* strains were grown on PDA in Petri dishes for two weeks at 25°C in the dark. Ten plates per each strain were used. Conidia were harvested by adding 20 ml of sterile distilled water and Tween 80 (0.01%) to each plate. Concentration was adjusted to 1 × 10⁶ conidia ml⁻¹ under a light microscope (Hund Wetzlar H 600LL, Wetzlar, Germany) by using Thoma-Zeiss counting chamber.

Plants bearing sporulating colonies of *P. xanthii* and *E. necator* were homogeneously treated with the suspension containing 1 × 10⁶ conidia ml⁻¹ of *A. quisqualis* by using a hand air-sprayer. Only two basal leaves per plant previously infected with the specific powdery mildew were treated (10 ml of suspension per leaf). On strawberry a drop of the suspension containing *A. quisqualis* conidia (10 µl each) was applied on powdery mildew spots of approximately 3 mm of diameter. Six spots per leaf were treated.

Plants with powdery mildew and *A. quisqualis* infections were kept at 25°C and 95 % of RH for 48 h and then stored at the same temperature and 70 ± 10% of RH. Untreated control plants were sprayed with distilled water and Tween 80 only. The reduction of sporulation of powdery mildews was evaluated by counting the number of *E. necator, P. aphanis* and P. *xanthii* conidia produced by the pathogen on the leaf surface 10 days after *A. quisqualis* treatment in comparison to untreated plants. For grapevine and cucumber, the two basal leaves of each plant were collected (12 leaves per strain) and leaf disks of 1.8 cm of diameter were cut (6 and 18 disks each grapevine and cucumber leaf, respectively). Leaf disks were transferred into 50 ml tubes with distilled water and Tween 80 (0.01%). Grapevine and cucumber leaf disks were washed and vortexed for 1 min into a volume of 5 and 15 ml, respectively. For strawberry, two leaves per plant were picked up and a leaf disk of three mm in diameter was cut from each lesion and transferred into a 2 ml tube with 1 ml of distilled water plus Tween 80 (0.01 %).

In the three pathosystems tubes were vortexed for 1 min and four droplets of 20 µl per leaf were mounted on glass slide. Concentration of powdery mildew conidia was measured under a light microscope. Conidia were counted using a Thoma-Zeiss counting chamber. Results were expressed as percentage reduction of the number of powdery mildew conidia in comparison with untreated control.

In vivo bioassays showed that all *A. quisqualis* tested strains were able to significantly reduce the sporulation of P. *xanthii, E. necator* and *P. aphanis* (Fig. 3) in comparison with untreated control. Interestingly, some differences in reducing sporulation were observed on different powdery mildew species. In all experiments, strain Fem307 isolated *from E. necator* on grape in Italy was more effective than the commercial strain AQ10. The same was true as to the strain ATCC 200245, where Fem307 was also significantly more effective (data not shown).

Strain Fem307 which is highly effective against one powdery mildew is also the best performing against the other two powdery mildews. Values of powdery mildew conidia reduction in comparison with untreated control ranged from 53% to 97%. Furthermore, powdery mildew species seem to have a different susceptibility versus *A. quisqualis.* In fact, experiments showed that *P. aphanis* is in general less susceptible to all the different A. *quisqualis* strains than *P. xanthii* and *E. necator.* The best performance was achieved through the application of strain Fem307 and AQ10 on grapevine plants (97 and 84%, respectively).

### Example 3: Preparation of an exemplary mushroom and shrimp shell preparation.

The following example provides an exemplary way of preparing a preferred mushroom preparation and preferred shrimp shell preparation.

Mushrooms and shrimps shells were dried and ground. Mushroom and shrimp shell pieces were first lysed mechanically using a tissue lyser (type MM200; Retsch, Germany). 2 g of these extracts were added in an Eppendorf tube containing 2 two stainless steel balls and shaken for 15 min at maximum frequency of 25 Hz to obtain a fine powder. Mushroom and shrimp shell powders were suspended in distilled water at the concentration of 1 g/L. (Alternatively, powders may e.g. also be suspended in saline solution.) Then, suspensions were preferably exposed at 121°C for 20 min (by autoclave), e.g. in order to disrupt big agglomerates. Finally, suspensions were filtered by using Whatman n. 4 filter paper disks to have homogenized mushroom and shrimp shell suspension and stored into 50 ml falcon tubes at 4°C for 30 days.

### Example 4: Activators enhancing the conidial germination of A. quisqualis

The present inventors thoroughly investigated the possibility of increasing the conidial germination rate of different *A. quisqualis* strains. Here, the strain Fem307 isolated from *Erysiphe necator* in Italy was used in comparison to e.g. the commercial AQ10 strain (AF035783) isolated from powdery mildew of *Chata Edulis* in Israel and an additional strain ATCC 200245 (AF126817) isolated from *Erysiphe necator* in USA and available in ATCC culture collection as additional strain. Fem307 has been isolated by the present inventors and has been deposited in context with the present invention (cf. above). Other strains were obtained from Intrachem Bio Italia (AQ10) and from culture collection (ATCC200245, ATCC, American Type Culture Collection, Rockville, MD, USA). The present inventors evaluated the effect of four different substances (Table 2) on the germination rates of three selected *A. quisqualis* strains. For the preparation of the powdery mildew extract, fresh colonies of cucumber powdery mildew *(P. xanthii)* were collected from leaf surface (10-15 leaves) by using 500 ml of distilled water + the adjuvant Tween 80 0.01%. Then, the spore concentration was adjusted by Thoma-Zeiss to 1x10⁵ conidia ml⁻¹. The suspension of distilled water + Tween 80 containing 1x10⁵ conidia ml⁻¹ of cucumber powdery mildew is filtered and warm up (65°C twice - pasteurization). Other substances were homogenized into distilled water at the concentration of 1g l⁻¹.

**Table 2 Substances used to stimulate the conidia germination of A. quisqualis strains**

| Substance | Active ingredient | Dosage |
|---|---|---|
| Powdery mildew | Powdery mildew extract | 1 x 10⁵ conidia ml⁻¹ |
| Shrimp Shell | Shrimp Shells powder | 1 g l⁻¹ |
| Beer yeast (fungus) | *Saccharomyces cerevisiae* cells | 1 g l⁻¹ |
| Mushroom | Mushroom powder | 1 g l⁻¹ |

In certain experiments, each strain was paired with the four selected substances, Sterile distilled water suspensions of the substances together with each fungal strain (1 × 10⁵ conidia ml⁻¹) were prepared. Suspension of only *A. quisqualis* conidia in sterile distilled water were used as controls. Four drops (10 µl each) per combination (four replicates) were put onto a glass slide, which was placed in a Petri dish (RH = 100%) and stored in a dark incubator kept at 25°C, which is the temperature that Gu and Ko (1997) used in their study of the factors affecting the germination of *A. quisqualis* conidia. Evaluation of the germination rates and germ-tube elongation of 200 conidia per strain (four replicates, 50 conidia per replicate) was carried out under a light microscope after 12 hours.

Cucumber *(Podosphaera xanthii),* strawberry *(Podosphaera aphanis)* and grapevine (*Erysiphe necator*) powdery mildew were used as fungal hosts to evaluate the ability of *A. quisqualis* in reducing mildew infections in controlled conditions. Two weeks old plants were inoculated with powdery mildew spraying a suspension of distilled water containing 1 × 10⁵ conidia ml⁻¹. Once powdery mildew cultures were sporulating (four/seven days later) a suspension of distilled water containing 1 × 10⁶ conidia ml⁻¹ of *A. quisqualis* and some soluble substances was prepared. Conidia of *A. quisqualis* were collected from five plates adding 20 ml of distilled water + Tween 80 and adjusting the concentration to 1 × 10⁶ conidia ml⁻¹. Then, a mixture of *A. quisqualis* conidia (250 ml) and soluble substances (250 ml, 1 g/l) was prepared. Flasks of 500 ml were used. Different soluble substances (powdery mildew extract, shrimp shell, mushroom, beer yeast) promoting the germination of *A. quisqualis* conidia were added to the conidial suspension 12 hours before inoculation time. Ten days after *A. quisqualis* infection the powdery mildew infected leaf area (severity) was evaluate by observing the presence of powdery mildew spots on the upper leaf surface.

Assays were performed on three independent experiments arranged in a randomized complete block design with three replications. Data were first tested for the homogeneity of variances and then subjected to the analysis of variance (ANOVA). Two-way ANOVA was performed on log-transformed data from the three independent experiments and revealed no significant Experiment × Treatment interaction. Therefore, data from the three experiments were pooled. Averages of all replicates are presented in Figure 4 and 5. Statistical significances among treatments were computed with Statistica software 7.0 (Statsoft, Tulsa, OK, USA) and means were separated using the Tukey's HSD (P < 0.05).

Physiological tests with different *A. quisqualis* strains showed that the germination rate and germ-tube length of *A. quisqualis* conidia increased greatly in the presence of different substances in accordance with the present invention. Most of the tested substances, except beer yeast, increased the germination rate and tube elongation of the *A. quisqualis* strains at least to a certain degree (Fig. 4). However, significant differences in the induction of germination were detected among the different substances. For example, the conidial germination and tube elongation of strains were stimulated more strongly by Shrimp shells (60.4 - 87 % and 19 - 30.5 um) and much less affected by Beer yeast (6.6 - 17.9 % and 5.4 - 6.9 um). The present inventors observed also differences among the conidial germination rates of the different strains, independent of the type of substance. With the exception of beer yeast, the germination rate of Fem307 was strongly induced by the tested substances (61.6 - 87 %) whereas AQ10 and ATCC 200245 were generally the least responsive. AQ10 exhibited significantly lower germination rates and less tube elongation in the presence of several substances stimulating conidia, as compared to the strain Fem307.

Notably, it can be concluded from the above experiments that there was a significant effect of boosting germination of *Ampelomyces quisqualis* by mushroom preparation and shrimp shell preparations. As opposed to that, fungal microorganisms as *Saccharomyces cerevisiae* (beer yeast) have almost no effect. A certain degree of activation of the target *(Ampelomyces quisqualis*), however, was expected in view of prior art studies. Importantly, the preparations of the present invention based on shrimp shells or mushrooms are strongly boosting germination of all the tested strains.

Moreover, experiments conducted on three different fungal hosts showed that the ability to reduce mildew infections of *A. quisqualis* strains can be increased by the presence of different substances with a stimulatory effect on conidia (Fig. 5). In the presence of these substances promoting conidial germination, all tested strains showed a stronger reduction of infected leaf area. However, individual strains differ in their effectiveness and Fem307 was more effective than ATCC 200245 and the commercial strain AQ10. Best results were obtained with Fem307 stimulated by shrimp shell and mushroom, which reduced the infected leaf area on strawberry (Fig. 5a), grapevine (Fig. 5b) and cucumber (Fig. 5c) (values ranged from 62 to 91 %). On the other hand, the results indicate there is no statistically significant difference between control (untreated conidia) and conidia stimulated by beer yeast on all tested plants. The improving of efficacy obtained with AQ10, Fem307 and ATCC 200245 was higher on strawberry powdery mildew which is less susceptible versus *A. quisqualis* than cucumber and grapevine. For example, Fem307 and AQ10 conidia stimulated by shrimp shell increased the reduction of mildew infections of more than 20 % in comparison to the control (Fig. 5a).

### References

Edgar RC (2004) MUSCLE: multiple sequence alignment with high accuracy and high throughput. Nucleic Acids Research 32:1792-1797
Gardes M and Bruns TD (1993) ITS primers with enhanced specificity for basidiomycetes - application to the identification of mycorrhizae and rusts. Molecular Ecology, 2, 113-118
Gilardi G, Baudino M, Garibaldi A, Gullino ML (2012) Efficacy of biocontrol agents and natural compounds against powdery mildew of zucchini. Phytoparasitica 40: 147-155
Gu YH and Ko WH (1997) Water agarose medium for studying factors affecting germination of conidia of Ampelomyces quisqualis. Mycological Research 101: 422-424
Kiss L (1997) Genetic diversity in Ampelomyces isolates, hyperparasites of powdery mildew fungi, inferred from RFLP analysis of the rDNA ITS region. Mycological Research 101: 1073-1080
Kiss L, Pintye A, Kovacs GM, Jankovics T, Fontaine MC, Harvey N, Xu X, Nicot PC, Bardin M, Shykoff JA, Giraud T (2011) Temporal isolation explains host-related genetic differentiation in a group of widespread mycoparasitic fungi. Molecular Ecology 20: 1492-1507
Liang C, Yang J, Kovacs GM, Szentivanyi O, Li B, Xu XM, Kiss L (2007) Genetic diversity of Ampelomyces mycoparasite isolated from different powdery mildew species in China inferred from analyses of rDNA ITS sequences. Fungal Diversity 24: 225-240
Park MJ, Choi YJ, Hong SB, Shin HD (2010) Genetic variability and mycohost association of Ampelomyces quisqualis isolates inferred from phylogenetic analyses of ITS rDNA and actin gene sequences. Fungal Biology 114: 235-247
Posada D and Crandall KA (1998) MODELTEST: testing the model of DNA substitution. Bioinformatics 14:817-818
Rotem Y, Yarden O, Sztejnberg A (1999) The mycoparasite Ampelomyces quisqualis expresses exgA encoding and exo-β-1,3 □-glucanase in culture and during mycoparasitism. Phytopathology 89: 631-638
Swofford DL (2002) PAUP*. Phylogenetic Analysis Using Parsimony (*and Other Methods). Version 4. Sinauer Associates, Sunderland, Massachusetts
Sztejnberg A (1993) Ampelomyces quisqualis AQ10, CNCM I-807, for biological control of powdery mildew. US patent no. 5190754
US5190754

Every citation of literature herein is explicitly incorporated by reference herein in its entirety.

## Claims

1. A fungal strain, selected from the group consisting of
i) *Ampelomyces quisqualis* Fem307,
ii) a strain obtainable from deposit CBS 126895,
iii) a strain comprising an ITS rDNA sequence that has at least 99.0 % pairwise identity to the ITS rDNA sequence of SEQ ID NO: 1, and
iv) a strain having an exgA gene that has at least 99.7 % pairwise identity to an exgA gene of a strain as defined in item i) or ii).

2. An fungal preparation, selected from the group consisting of
i) a preparation of *Ampelomyces quisqualis* Fem307,
ii) a preparation of a fungus obtainable from deposit CBS 126895,
iii) a preparation of a fungus comprising an ITS rDNA sequence that has at least 99.0 % pairwise identity to the ITS rDNA sequence of SEQ ID NO: 1, and
iv) a preparation of a fungus having an exgA gene that has at least 99.7 % pairwise identity to an exgA gene of a fungus as defined in item i) or ii);
particularly wherein said fungal preparation is a biofungicide, especially a biofungicide against powdery mildews in plants.

3. Use of a fungal strain according to claim 1 or of a fungal preparation according to claim 2 for treating powdery mildews in plants.

4. A method of treating powdery mildews in plants, comprising a step of contacting the plant to be treated with a fungal strain according to claim 1 or with a fungal preparation according to claim 2.

5. The use of claim 3 or the method of claim 4, additionally comprising adding
i) a mushroom preparation, particularly from *Agaricus bisporus,* and/or
ii) a shrimp shell preparation.

6. The use or method of any one of claims 3 to 5, wherein said fungal strain or fungal preparation has improved biocontrol efficacy and/or fungicide efficacy as compared to a strain or preparation of *Ampelomyces quisqualis* AQ10.

7. Use of
i) a mushroom preparation, particularly from *Agaricus bisporus,* and/or
ii) a shrimp shell preparation,
in treating powdery mildews in plants with *Ampelomyces quisqualis..*

8. A method of treating powdery mildews in plants with Ampelomyces quisqualis, the method comprising a step of adding to *Ampelomyces quisqualis*
i) a mushroom preparation, particularly from *Agaricus bisporus,* and/or
ii) a shrimp shell preparation.

9. The use of claim 7 or the method of claim 8, wherein said *Ampelomyces quisqualis* is a fungal strain according to claim 1 or a fungal preparation according to claim 2.

10. The use of claim 7 or the method of claim 8, wherein said *Ampelomyces quisqualis* is AQ10.

11. The use or method of any one of claims 7 to 10, wherein said mushroom preparation and/or shrimp shell preparation
i) improve(s) germination of *Ampelomyces quisqualis* conidia, and/or
ii) enhance(s) biocontrol efficacy of the *Ampelomyces quisqualis,* and/or
iii) enhance(s) fungicide efficacy of the *Ampelomyces quisqualis.*

12. The use or method of any one of claims 3 to 11 wherein said powdery mildews are treated with a suspension of *Ampelomyces quisqualis* conidia.

13. The use or method of any one of claims 5 to 12 wherein
i) said mushroom preparation is a suspension of a powder derivable from an edible mushroom, particularly from *Agaricus bisporus,* and/or
ii) wherein said shrimp shell preparation is a suspension of a powder derivable from shrimp shells.

14. Use of i) a mushroom preparation, particularly from *Agaricus bisporus,* and/or ii) a shrimp shell preparation, for enhancing biocontrol efficacy and/or fungicide efficacy of *Ampelomyces quisqualis,*
particularly wherein said mushroom preparation and/or shrimp shell preparation and/or said *Ampelomyces quisqualis* is further defined as in any of the preceding claims.

15. A composition comprising a) an activator of *Ampelomyces quisqualis,* selected from a mushroom preparation, a shrimp shell preparation and a mixture thereof, and b) a fungal strain or a fungal preparation of *Ampelomyces quisqualis,* in particular the fungal strain or fungal preparation according to claims 1 or 2.


SEQUENCE LISTING

<110>  Fondazione Edmund Mach

<120>  Novel Treatments of Powdery Mildews in Plants

<130>  M68205EP

<150>  IT VI2012A000228
<151>  2012-09-19

<160>  10

<170>  PatentIn version 3.5

<210>  1
<211>  501
<212>  DNA
<213>  Ampelomyces quisqualis

<400>  1
cttccgtagg tgaacctgcg gaaggatcat tatcttcagt actttgctac tgacaggtgg      60

gcctgttagc ctgtatagta ttactactga tgagcagcat ggccacagcc cttacccttg     120

tcttttgtgc acctatgttt ccatagcagg ctgacaacct tgctggacaa ccccatacca     180

tgaatgtttt atcagcgtct gaaaaaccta aaatttacaa ctttcaacaa cggatctctt     240

ggttctggca tcgatgaaga acgcagcgaa atgcgataag tagtgtgaat tgcagaattc     300

agtgaatcat cgactctttg aacgcacatt gcgccccttg gtattccatg gggcatgcct     360

gttcgagcgt catttgtacc tcaagctatg cttggtgttg ggtgcctgtc cttgttcttt     420

cttggaccca ccttaaagca attggcagcc agtgttttgg tatagaactg cagcacattt     480

aacaattcta gtaccaaaga c                                               501


<210>  2
<211>  2334
<212>  DNA
<213>  Ampelomyces quisqualis

<400>  2
atgcttgcct tcagcgctgg tgcgctcctt ctgacgctgc gcgtcttcct cgccgcgact      60

ccatcggccg cagcacctgt cgcccaagca gcagaggtac cgcaggccgg cgcctctggc     120

tattggttcg gcaacatcaa gcgccagggt atcgctccat acaatgagaa tcccgctgcc     180

tacaaggttt tccgcaacgt caagtcactt ggagccaaag gcgacggcat taccgatgac     240

accgctgcca tcaatgcggc cattgcggac ggcaaccgtt gtggtcaagg ctgcgactca     300

acaaccacta gcccagccat catctacttc cccgctggta catacttgat cagcgagccc     360

atcatccagt actactacac ccagttcgtc ggtgatgcga ccaacccgcc tacactgaag     420

gctaagaaca ctttcgaggg catgggcttg atcgatgcgg acccctatat ccccggcggc     480

gacggcgcca actggtacac caaccagaac aacttttacc gccagattcg taacttcgtc     540

atcgacatca aggataccaa ggccgctgct ggcattcact ggcaggtgtc tcaggccact     600

tctctccaga acatccgctt cgagatggcc acgggcgagg ccggtgcgaa ccagaaaggc 660

attttccaag acaatggctc cggtggtttc atgtctgatc tcgtcttcaa tggtggtgct 720

atcggcgcct tcctcggttc ccagcagttc accacgcgca acatgacctt caacaactgc 780

ggcactgcca tcttcatgaa ctggaactgg ctgtggaccc tgaagagcat cttcatcaac 840

gactgcaagc tgggtctcga catggctaac agccccaaca accaaactgt cggctctgtc 900

ctccttctcg acagcaaatt caccaacacc cccatcggca tcaactcttc tttcacccaa 960

gatagcgtcc cccacaccgg cggtactctc attattgaca acgtcgactt cgagggtagc 1020

aatgtggctg tccagaacgt cgccggcgag actctccttg ctggtaagac taaggtcgcc 1080

acctgggccc aaggtaatgc catggctgcc ggtcaggctc aagctggtcg tgtccaaggc 1140

gatgtcaaca acccacctac caagccacag agtcttctcg gcgaaaatgg ctggttcgag 1200

cgtagcaagc cccagtacga gaacgtcgac gtgtccaagt ttgtcagtct caagggcgca 1260

ggtgcggtgg gcgacggtgt caccgatgat actgccatga ttcagaaagc catcgatggt 1320

ctccaagacg gccagatcct gcatgctgac cacggcgcct acctcattac caagactatt 1380

gaaatccctg cggagaagaa tatcaagatt gtcggcgaga tctacaccat gtttttcatt 1440

actggcaagt tcttcggcaa catggacgac cccagcctgg gattccgtgt cggcaagaag 1500

agcggtgaca agggtacttt cgagatgtcc gacgccatca tcagcactca gggtcctgca 1560

cctggaggta tcttgatgga gtggaatatc aatgccgagg ctggcaaggc cggtctttgg 1620

gacgtccact tccgtgttgg cggtttcgca ggcaccaatc tacagtcttc caactgcaag 1680

aagaaccccg acacggagca tcctcccaac gaagagtgta ttggttcctt catgcagctc 1740

cacattacca agagctccag cggctacttt gagaacgtct ggctttggac cgctgaccac 1800

gagctcgacc agcctgacca cgcacaaatc gacatttaca acggtcgtgg catgctcgtt 1860

gaatcccaag gtccagtctg gcttgtcggt accgcatccg agcactctca actttctcag 1920

taccaattcc aaggtgccaa ggacatttgg tacggcgcca ttcagaccga aactccctac 1980

taccagccca accccaaagc aaacgtgccc ttcaagaaga acgacaagtt ctttgaccca 2040

gacatgagca acaccacctc ggcctgggcg gttcgcatca tcgatagctc aagcatttgg 2100

aactacggtg ctggaaccta ctccttcttc gacaattact cgcaaaagtg cgttgtgggc 2160

cagaactgcc aggagcacat caacgagatc gagaactcca gaaacgtcaa catctttggt 2220

ctctcgacca aggcgtctgt caacatgatc tcatctggtg gtgtaggctt ggtaaaggac 2280

gaggacaatc ggtcaaactt ttgtgctacg ctgggtattt ttgctcaggc atag 2334

<210> 3
<211> 19
<212> DNA
<213> Artificial Sequence

<220>
<223>  primer

<400>  3
tccgtaggtg aacctgcgg                                                    19


<210>  4
<211>  20
<212>  DNA
<213>  Artificial Sequence

<220>
<223>  primer

<400>  4
tcctccgctt attgatatgc                                                   20


<210>  5
<211>  24
<212>  DNA
<213>  Artificial Sequence

<220>
<223>  primer

<400>  5
ccgcaactca agcattccaa caaa                                              24


<210>  6
<211>  24
<212>  DNA
<213>  Artificial Sequence

<220>
<223>  primer

<400>  6
tgctaataaa aggccctctc atca                                              24


<210>  7
<211>  20
<212>  DNA
<213>  Artificial Sequence

<220>
<223>  primer

<400>  7
tcggcgcctt cctcggatct                                                   20


<210>  8
<211>  21
<212>  DNA
<213>  Artificial Sequence

<220>
<223>  primer

<400>  8
cgagacccag cttgcagtcg t                                                 21

<210>  9
<211>  20
<212>  DNA
<213>  Artificial Sequence

<220>
<223>  primer

<400>  9
ggacgacccc cagcctggat                                                    20


<210>  10
<211>  20
<212>  DNA
<213>  Artificial Sequence

<220>
<223>  primer

<400>  10
ggccttgcca gcctcagcat                                                    20